(19) **Europäisches Patentamt**

**European Patent Office**

**Office européen des brevets**

(11) **EP 1 258 721 B1**

(12) **EUROPÄISCHE PATENTSCHRIFT**

(45) Veröffentlichungstag und Bekanntmachung des Hinweises auf die Patenterteilung:
**08.02.2006 Patentblatt 2006/06**

(51) Int Cl.:
*G01N 9/26* *(2006.01)* *G01N 7/14* *(2006.01)*

(21) Anmeldenummer: **02010379.2**

(22) Anmeldetag: **08.05.2002**

(54) **Verfahren zur Bestimmung des Gasgehaltes eines Fluids**

Method for determining the gas content of a fluid

Procédé pour déterminer le contenu gazeux d'un fluide

(84) Benannte Vertragsstaaten:
**AT DE FI IT SE**

(30) Priorität: **15.05.2001 DE 10123530**

(43) Veröffentlichungstag der Anmeldung:
**20.11.2002 Patentblatt 2002/47**

(73) Patentinhaber: **Voith Paper Patent GmbH**
**89522 Heidenheim (DE)**

(72) Erfinder:
• **Schwarz, Michael, Dr.**
**89522 Heidenheim (DE)**
• **Keim, Volker**
**Bangkok 10110 (TH)**

(56) Entgegenhaltungen:
EP-A- 0 790 545         DE-A- 10 043 142
DE-A- 10 120 885      FR-A- 2 687 475
GB-A- 1 071 798        US-A- 4 238 208
US-A- 4 478 615        US-A- 4 581 934
US-A- 5 731 518

EP 1 258 721 B1

**Beschreibung**

Die Erfindung betrifft ein Verfahren zur Regelung des Gasgehaltes eines bei der Herstellung einer Faserstoffbahn, insbesondere Papier- oder Kartonbahn, eingesetzten oder anfallenden Fluids.

An allen schnelllaufenden Papiermaschinen wird die Suspension vor dem Stoffauflauf mechanisch entlüftet, wodurch der Gasgehalt im Stoffauflauf annähernd vollständig entfernt wird. Dennoch müssen dem Siebwasser zusätzlich chemische Entlüfter- oder Entschäumermittel zugegeben werden, um Förderprobleme zu vermeiden. Die zugegebene Entlüfter- bzw. Entschäumermenge wird entweder konstant dosiert oder über Online-Luftgehaltsmessungen geregelt. Dabei ist in der Regel ein sogenannter Batch-Betrieb, d.h. eine diskontinuierliche Messung vorgesehen. Daraus ergeben sich nun aber u.a. die folgenden Nachteile:

Wird konstant dosiert, so muß die Entlüfter- bzw. Entschäumermenge so großzügig gewählt werden, daß auch im ungünstigsten Fall Störungen vermieden werden. Da die Kosten von Additiven generell hoch sind, stellt diese Überdosierung einen wichtigen Kostenfaktor dar. Wird der Gasgehalt online gemessen und damit die Entlüfter- bzw. Entschäumermenge minimiert, so ist neben den hohen Investitionskosten für das betreffende Gerät auch für die Wartung ein beträchtlicher Aufwand erforderlich.

Die US 4,581,934 offenbart ein Verfahren zur Messung des Gasgehalts in einer Flüssigharzkomponente zur Herstellung von Polyurethanschaum, wobei der Gasgehalt über den hydrostatischen Druck einer aus der Flüssigharzkomponente aufgebauten Säule gemessen wird.

Ziel der Erfindung ist es, ein verbessertes Verfahren der eingangs genannten Art anzugeben, mit der auf zuverlässige und wirtschaftliche Weise insbesondere auch eine kontinuierliche Gasgehaltsbestimmung möglich ist.

Diese Aufgabe wird erfindungsgemäß gelöst durch ein Verfahren mit den Merkmalen des Anspruchs 1.

Mit dieser Ausgestaltung lassen sich Chemikalien einsparen. Es ist eine geregelte Dosierung der Additive auch ohne teures Meßgerät möglich. Der betreffende Gasgehalt läßt sich insgesamt einfacher, schneller und kostengünstiger ermitteln, wobei die gewünschte Information über den Gasgehalt kontinuierlich in Echtzeit erhalten wird. Die Zugabe von Additiven kann jederzeit an sich ändernde Prozeßbedingungen angepaßt werden.

Vorzugsweise wird die Temperatur, insbesondere die Temperatur in der Fluidsäule, bei der Regelung des Gasgehalts mit berücksichtigt. Dabei kann die Temperatur beispielsweise konstant gehalten werden.

Gemäß einer zweckmäßigen Ausgestaltung des erfindungsgemäßen Verfahrens wird als Referenzdruck der hydrostatische Druck einer Fluidsäule ohne Gasgehalt herangezogen. Damit ist eine absolute Gasgehaltsmessung bzw. -bestimmung möglich. Es kann also bestimmt werden, wieviel Luftmasse (Gramm) pro Fluidmasse (Gramm) enthalten ist. Dabei kann bei der Regelung des Gasgehaltes insbesondere auch die Temperatur der den Referenzdruck liefernden Fluidsäule mit berücksichtigt werden.

Der Referenzdruck kann beispielsweise in einem Regler oder dergleichen als feste Größe gespeichert und/oder bereitgestellt werden.

Gemäß einer vorteilhaften Ausgestaltung des erfindungsgemäßen Verfahrens wird als Referenzdruck der hydrostatische Druck der Fluidsäule bezüglich deren Zustandes zu einem früheren Zeitpunkt herangezogen. Die Auswertung ergibt dann eine relative Änderung des Gas- bzw. Luftgehaltes. Der Gasgehalt des Fluides ändert sich zeitlich. Grundsätzlich kann zu verschiedenen Zeiten auch ein unterschiedlicher Referenzdruck vorliegen.

Das erfindungsgemäße Verfahren ist vorteilhafterweise in wenigstens einem Regelungssystem einer Anlage zur Herstellung einer Faserstoffbahn, insbesondere Papier- oder Kartonbahn, anwendbar.

Von Vorteil ist insbesondere auch eine Anwendung des erfindungsgemäßen Verfahrens im konstanten Teil, d.h. im Stoffzuführsystem, einer Anlage zur Herstellung einer Faserstoffbahn, insbesondere Papier- oder Kartonbahn.

Dabei kann die Gasgehaltsregelung eingesetzt werden, bei der der Gasgehalt insbesondere über die variable Dosierung einer Entschäumer- und/oder Entlüfterzugabe entsprechend nachstellbar ist.

Mittels des erfindungsgemäßen Verfahrens kann beispielsweise im Siebwasser das Niveau mittels Drucktransmitter oder -sensoren gemessen werden. Ändert sich der Luftgehalt im Siebwasser, so ändert sich aufgrund der unterschiedlichen Dichte auch die Transmitteranzeige, ohne daß das tatsächliche Niveau eine Änderung erfährt. Da der Siebwasser-Turm normlerweise mit Überlauf gefahren wird, d.h. stets ein konstantes Niveau besitzt, kann die Transmitteranzeige als indirekte Luftgehaltsmessung insbesondere zur Regelung herangezogen werden.

Der Gasgehalt im jeweiligen Fluidzulauf kann beispielsweise über ein Gerät wie insbesondere das Gasanalysegerät "GAS 60" der Firma Mütek Analytic GmbH, DE 82211 Herrsching, bestimmt werden. Bei diesem Gerät fließt die betreffende Probe kontinuierlich durch die Meßzelle der Einheit. Sobald der Meßzyklus beginnt, unterbrechen Einlaß- und Auslaßventile den Fluß. Die in der Meßzelle eingeschlossene Probe wird mittels eines Kolbens komprimiert und dann expandiert. Dabei wird der absolute Gasgehalt der Probe auf der Basis des idealen Gasgesetzes bestimmt. Bevor mit einer neuen Messung begonnen wird, wird die Meßzelle mit Wasser ausgespült.

Die Erfindung wird im folgenden anhand von Ausführungsbeispielen unter Bezugnahme auf die Zeichnung näher erläutert; in dieser zeigen:

Figur 1     eine schematische Teildarstellung eines beispielhaften Regelungssystems zur Regelung des Gasgehalts in einer Fluidsäule, in dem zur Bestimmung des jeweiligen Gasgehalts mittels eines Drucksensors der hydrostatische Druck in der Fluidsäule gemessen wird, und

Figur 2     ein Zeitdiagramm, aus dem sich ein anhand von praktischen Versuchsergebnissen erhaltener Zusammenhang zwischen dem hydrostatischen Druck und dem Gasgehalt einer Fluidsäule ergibt.

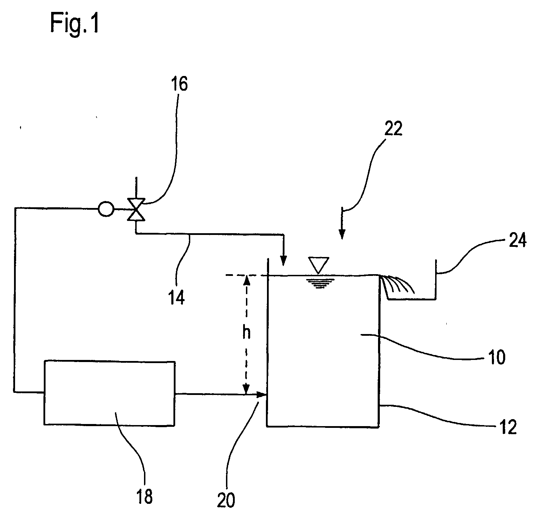

Figur 1 zeigt in schematischer Teildarstellung ein rein beispielhaftes Regelungssystem zur Regelung des Gasgehalts in einer Fluidsäule 10, die beispielsweise durch Siebwasser gebildet sein kann, das in einem Siebwasserbehälter 12 enthalten ist, der in der Naßpartie einer entsprechenden Papiermaschine anfallendes Siebwasser aufnimmt. Bei dieser Gasgehaltsregelung ist der Gasgehalt beispielsweise über die variable Dosierung einer Entschäumer- und/oder Entlüfterzugabe entsprechend nachstellbar. Die betreffenden Entschäumer- bzw. Entlüftermittel können beispielsweise über eine Leitung 14 zugeführt werden, in der ein entsprechend einstellbares Ventil 16 angeordnet ist.

Im vorliegenden Fall wird das Ventil 16 durch einen Regler 18 angesteuert, mit dem ein Drucksensor 20 verbunden ist, über den der hydrostatische Druck der Fluidsäule 10 gemessen wird. Über den Zulauf 22 wird dem Behälter 12 neues Fluid, d.h. hier beispielsweise Siebwasser, zugeführt. Dem Behälter 12 ist ein Überlauf 24 zugeordnet, in dem aus dem Behälter 12 überlaufendes Fluid eintritt, so daß insbesondere die ab dem Drucksensor 20 gemessene Höhe h der Fluidsäule 10 konstant gehalten werden kann.

Zur Bestimmung des Gasgehaltes in der Fluidsäule 10 wird also zunächst mittels des Drucksensors 20 der hydrostatische Druck der Fluidsäule 10 gemessen. Dabei wird die Höhe h der Fluidsäule 10 konstant gehalten. Der mittels des Drucksensors 20 erhaltene Meßwert wird im vorliegenden Fall dem Regler 18 zugeführt, in dem der gemessene hydrostatische Druck dann mit einem Referenzdruck verglichen wird. Beispielsweise in dem Regler 18 erfolgt dann eine entsprechende Auswertung des Vergleichsergebnisses bzw. der sich ergebenden Druckdifferenz. Anhand des Vergleichsergebnisses kann dann der tatsächliche Gasgehalt bestimmt werden.

Ist der bestimmte tatsächliche Gasgehalt zu hoch oder zu niedrig, so kann der gewünschte Gasgehalt beispielsweise über eine entsprechende Dosierung der Entschäumer- und/oder Entlüfterzugabe, d.h. durch eine entsprechende Beaufschlagung des Ventils 16, in der erforderlichen Weise nachgestellt werden. Im vorliegenden Fall ist also der Gasgehalt die Regelgröße, die über eine entsprechende Variation der Dosierung der Entschäumer- bzw. Entlüfterzugabe im Sinne einer Angleichung an den Referenzdruck nachgestellt wird.

Die Temperatur, insbesondere die Temperatur in der Fluidsäule 10, kann bei der Bestimmung des Gasgehaltes mit berücksichtigt werden. Dabei kann diese Temperatur insbesondere konstant gehalten werden.

Als Referenzdruck kann beispielsweise der hydrostatische Druck einer Fluidsäule ohne Gasgehalt herangezogen werden. Damit ist eine absolute Gasgehaltsmessung möglich. Es kann also bestimmt werden, welche Luftmasse (z.B. in Gramm) in einer bestimmten Fluidmasse (z.B. ein Gramm) enthalten ist.

Für den beispielsweise in der Einheit "Pa" angebbaren Referenzdruck $P_R$ gilt die Beziehung

$$(1) \qquad P_R = \rho_{F_0} \cdot g \cdot h,$$

wobei $\rho_{F_0}$ = Dichte des Fluids $[\frac{kg}{m^3}]$, g = Erdbeschleunigung $[\frac{m}{s^2}]$ und h = Höhe der Fluidsäule 10 über dem Drucksensor 20 [m]. Dieser Referenzdruck kann als feste Größe beispielsweise im Regler 18 gespeichert bzw. bereitgestellt sein. Dabei kann die betreffende Temperatur mit berücksichtigt werden.

Als Referenzdruck kann beispielsweise auch der hydrostatische Druck der Fluidsäule bezüglich deren Zustandes zu einem früheren Zeitpunkt herangezogen werden. Die Auswertung ergibt dann eine relative Änderung des Gas- bzw. Luftgehaltes. Der Gasgehalt des Fluides ändert sich zeitlich. Auch der Referenzdruck kann zu verschiedenen Zeiten unterschiedlich sein, d.h. sich mit der Zeit ändern.

Wird also zu einem früheren Zeitpunkt t - 1 der hydrostatische Druck der Fluidsäule 10 gemessen und dieser Druck als Referenzdruck herangezogen, so ergibt sich für diesen Referenzdruck $P_R$ gemäß der Beziehung (1):

$$(2) \qquad P_R(t - 1) = \rho_{FG}(t - 1) \cdot g \cdot h,$$

wobei $\rho_{FG}(t - 1)$ = Dichte des Gas enthaltenden Fluides zum Zeitpunkt t-1.

Für den zum späteren Zeitpunkt t gemessenen hydrostatischen Druck der Fluidsäule 10 gilt:

$$(3) \qquad P(t) = \rho_{FG}(t) \cdot g \cdot h$$

Zur Bestimmung der Gasgehaltsänderung wird die Dichteänderung ermittelt:

$$(4) \qquad P(t) - P(t-1) = (\rho_{FG}(t) - \rho_{FG}(t-1)) \cdot g \cdot h,$$

woraus folgt:

$$(5) \qquad \rho_{FG}(t) - \rho_{FG}(t-1) = \frac{P(t) - P(t-1)}{g \cdot h}$$

Die Gasgehaltsänderung wird dann aus dem Zusammenhang

$$(6) \qquad \rho_{FG} = f(\text{Gasgehalt } X_{Gas})$$

kann beispielsweise über ein Gasanalysegerät "GAS 60" der Firma Mütek Analytic GmbH, D-82211 Herrsching, erfolgen.

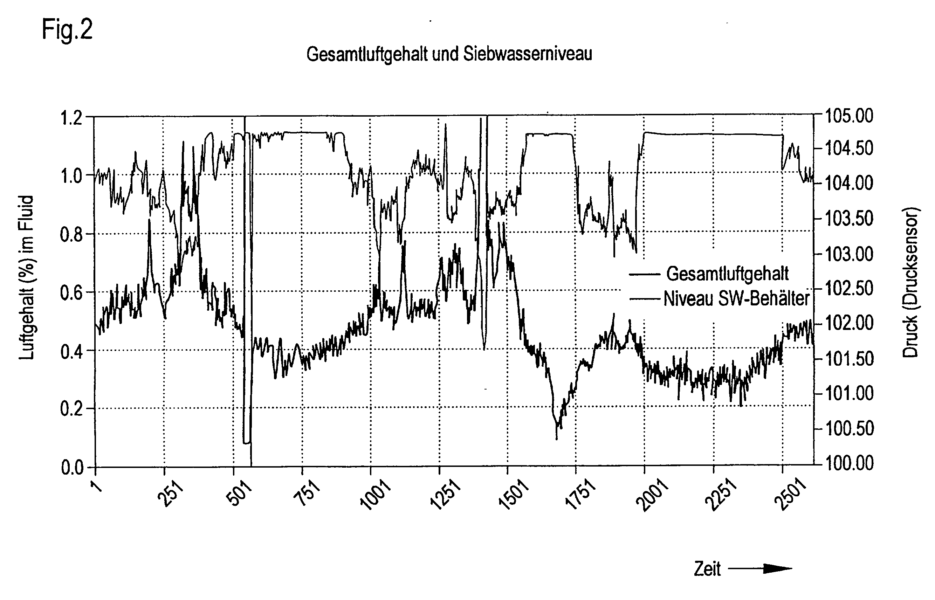

Aus dem in der Figur 2 dargestellten Zeitdiagramm ergibt sich ein anhand von praktischen Versuchsergebnissen erhaltener Zusammenhang zwischen dem hydrostatischen Druck und dem Gasgehalt einer Fluidsäule. Der betreffende Zusammenhang kann dann bei der Bestimmung des tatsächlichen Gas- bzw. Luftgehalts entsprechend berücksichtigt werden.

Dieser Zusammenhang kann berechnet oder aus experimentell bestimmten Tabellen entnommen werden.

Dabei ist bei einer jeweiligen Messung die Temperatur T des Fluids entweder konstant, oder sie ist bei der Bewertung zu berücksichtigen, da die Dichte von dieser Temperatur abhängig ist, d.h. $\rho = f(T)$ gilt.

Das beschriebene Verfahren zur Gasgehaltsbestimmung kann allgemein beispielsweise zu Regelungszwecken angewendet werden. So ist es beispielsweise in wenigstens einem Regelungssystem der in der Figur 1 angedeuteten Art einsetzbar.

Das angegebene Verfahren zur Gasgehaltsregelung kann insbesondere zur Luftgehaltsbestimmung im konstanten Teil einer Papiermaschine angewendet werden.

Bei dem weiter in Zusammenhang mit der Figur 1 beschriebenen Ausführungsbeispiel wird die beschriebene Gasgehaltsregelung eingesetzt, bei der der Gasgehalt über die variable Dosierung einer Entschäumer- und/oder Entlüfterzugabe entsprechend nachstellbar ist. Bei der Darstellung gemäß Figur 1 wird das Entschäumer- bzw. Entlüftermittel von oben her in den Behälter 12 gegeben. Grundsätzlich kann die Entschäumer- und/oder Entlüfterzugabe jedoch auch an einer anderen Stelle erfolgen.

Es ist beispielsweise auch eine Gasgehaltsbestimmung im Zulauf 22 (vgl. Figur 1) möglich. Eine solche Gasgehaltsbestimmung im Fluidzulauf 22

**Bezugszeichenliste**

10   Fluidsäule
12   Siebwasserbehälter
14   Leitung
16   Ventil
18   Regler
20   Drucksensor
22   Fluidzulauf
24   Überlauf

h     ab dem Drucksensor gemessene Höhe der Fluidsäule

**Patentansprüche**

**1.** Verfahren zur Regelung des Gasgehaltes eines bei der Herstellung einer Faserstoffbahn, insbesondere Papier- oder Kartonbahn, eingesetzten oder anfallenden Fluids, mit den folgenden Verfahrensschritten:

a) es wird der hydrostatische Druck einer das betreffende Fluid enthaltenden Fluidsäule (10) gemessen,
b) wobei die Höhe der Fluidsäule (10) konstant gehalten wird,
c) der gemessene Druck wird mit einem Referenzdruck verglichen, und
d) anhand des Vergleichsergebnisses bzw. durch eine entsprechende Auswertung dieses Vergleichsergebnisses wird der Gasgehalt bestimmt.
e) Zugabe von Entschäumer-und/oder Entlüftermittel zum Fluid abhängig vom bestimmten Gasgehalt zur Nachstellung des Gasgehalts im Fluid.

**2.** Verfahren nach Anspruch 1,
**dadurch gekennzeichnet,**
**daß** die Temperatur, insbesondere die Temperatur in der Fluidsäule (10), bei der Bestimmung des Gasgehaltes mit berücksichtigt wird.

**3.** Verfahren nach Anspruch 2,
**dadurch gekennzeichnet,**
**daß** die Temperatur konstant gehalten wird.

**4.** Verfahren nach einem der vorhergehenden Ansprüche,
**dadurch gekennzeichnet,**
**daß** als Referenzdruck der hydrostatische Druck einer Fluidsäule (10) ohne Gasgehalt herangezogen wird.

**5.** Verfahren nach Anspruch 4,
**dadurch gekennzeichnet,**
**daß** die Temperatur der den Referenzdruck liefernden Fluidsäule (10) bei der Bestimmung des Gasgehaltes mit berücksichtigt wird.

**6.** Verfahren nach einem der vorhergehenden Ansprüche,
**dadurch gekennzeichnet,**
**daß** der Referenzdruck insbesondere in einem Regler (18) oder dergleichen als feste Größe gespeichert und/oder bereitgestellt wird.

**7.** Verfahren nach einem der vorhergehenden Ansprüche,
**dadurch gekennzeichnet,**
**daß** als Referenzdruck der hydrostatische Druck der Fluidsäule (10) bezüglich deren Zustandes zu einem früheren Zeitpunkt herangezogen wird.

**8.** Verfahrens nach einem der Ansprüche 1 bis 7 **dadurch gekenneichnet dass** der Gasgehalt, insbesondere Luftgehaltes, im konstanten Teil einer Anlage zur Herstellung einer Faserstoffbahn, insbesondere Papier- oder Kartonbahnbestimmt wird.

**9.** Verfahren nach Anspruch 8,
**dadurch gekennzeichnet,**
**dass** der Gasgehalt des Siebwassers, insbesondere im Siebwasserbehälter (12), bestimmt wird.

**Claims**

**1.** Method for regulating the gas content of a fluid used or arising during the production of a fibrous web, in particular a paper or board web, having the following method steps:

a) the hydrostatic pressure of a fluid column (10) containing the relevant fluid is measured,
b) the height of the fluid column (10) being kept constant,
c) the measured pressure is compared with a reference pressure, and
d) by using the result of the comparison or by means of an appropriate evaluation of this result of the comparison, the gas content is determined,
e) addition of defoamer and/or deaerating agent to the fluid, depending on the gas content determined, in order to readjust the gas content of the fluid.

2. Method according to Claim 1, **characterized in that** the temperature, in particular the temperature in the fluid column (10), is also taken into account when determining the gas content.

3. Method according to Claim 2, **characterized in that** the temperature is kept constant.

4. Method according to one of the preceding claims, **characterized in that** the reference pressure used is the hydrostatic pressure of a fluid column (10) without any gas content.

5. Method according to Claim 4, **characterized in that** the temperature of the fluid column (10) supplying the reference pressure is also taken into account when determining the gas content.

6. Method according to one of the preceding claims, **characterized in that** the reference pressure is stored and/or provided as a fixed variable, in particular in a controller (18) or the like.

7. Method according to one of the preceding claims, **characterized in that** the reference pressure used is the hydrostatic pressure of the fluid column (10) with respect to its state at an earlier time.

8. Method according to one of Claims 1 to 7, **characterized in that** the gas content, in particular air content, is determined in the approach system of an installation for producing a fibrous web, in particular a paper or board web.

9. Method according to Claim 8, **characterized in that** the gas content is determined in the white water, in particular in the white water tank (12).

**Revendications**

1. Procédé pour réguler la teneur en gaz d'un fluide utilisé ou se formant lors de la fabrication d'une bande fibreuse, notamment d'une bande de papier ou de carton, comprenant les étapes de procédé suivantes :

a) la pression hydrostatique d'une colonne de fluide (10) contenant le fluide concerné est mesurée,
b) la hauteur de la colonne de fluide (10) étant maintenue constante,
c) la pression mesurée est comparée avec une pression de référence, et
d) la teneur en gaz est déterminée à l'aide du résultat de la comparaison ou par une analyse correspondante de ce résultat de la comparaison,
e) ajout d'agents anti-mousse et/ou d'agents de désaérage au fluide en fonction de la teneur en gaz déterminée en vue du rattrapage de la teneur en gaz dans le fluide.

2. Procédé selon la revendication 1,
**caractérisé en ce que**
la température, notamment la température dans la colonne de fluide (10), est prise en compte lors de la détermination de la teneur en gaz.

3. Procédé selon la revendication 2,
**caractérisé en ce que**
la température est maintenue constante.

4. Procédé selon l'une quelconque des revendications précédentes,
**caractérisé en ce que**
l'on utilise comme pression de référence la pression hydrostatique d'une colonne de fluide (10) sans teneur en gaz.

**5.** Procédé selon la revendication 4,
**caractérisé en ce que**
la température de la colonne de fluide (10) fournissant la pression de référence est prise en compte lors de la détermination de la teneur en gaz.

**6.** Procédé selon l'une quelconque des revendications précédentes,
**caractérisé en ce que**
la pression de référence est mémorisée et/ou mise à disposition notamment dans un régulateur (18) ou similaire sous forme de valeur fixe.

**7.** Procédé selon l'une quelconque des revendications précédentes,
**caractérisé en ce que**
l'on utilise comme pression de référence la pression hydrostatique de la colonne de fluide (10) par rapport à son état à un instant précédent.

**8.** Procédé selon l'une quelconque des revendications 1 à 7, **caractérisé en ce que** la teneur en gaz, notamment la teneur en air, est déterminée dans la partie constante d'une installation de fabrication d'une bande fibreuse, notamment d'une bande de papier ou de carton.

**9.** Procédé selon la revendication 8,
**caractérisé en ce que**
la teneur en gaz de l'eau blanche, notamment dans le récipient d'eau blanche (12), est déterminée.

## Fig.1

Fig.2